# EUROPEAN PATENT APPLICATION

(11) **EP 2 526 862 A1**
(43) Date of publication of application: **28.11.2012**
(21) Application number: 12168608.3
(22) Date of filing: 21.05.2012
(51) Int. Cl.: A61B 5/0484, A61B 5/055

(54) **Brain function measuring apparatus and brain function measuring method**

(30) Priority: 24.05.2011 JP 2011115521
(71) Applicant: Canon Kabushiki Kaisha, Tokyo 146-8501 (JP)
(72) Inventor: Ichimura, Yoshikatsu, Tokyo, Tokyo 146-8501 (JP)
(74) Representative: TBK

(57) **Abstract**

A brain function activity of a subject is measured using a brain function measuring unit by presenting multiple stimulations, which are adjusted to have no cognitive meaning when presented in a separate manner and to have a cognitive meaning when presented simultaneously as a combined stimulation, to the subject as the combined stimulation at regular intervals with a control of a control unit, thus measuring a higher cognitive brain activity responding to the combined stimulation separately from a lower cognitive brain activity.

## Description

### BACKGROUND OF THE INVENTION

### Field of the Invention

The present invention relates to a brain function measuring apparatus and a brain function measuring method, and more particularly, to a brain function measuring apparatus and a brain function measuring method for detecting response and function of a brain when a sensory stimulus is presented to a subject.

### Description of the Related Art

In fields of medical science, biology, and psychology, in order to study a brain function of a subject, an apparatus is employed, which presents various stimuli such as visual, haptic, and auditory stimuli to a subject and measures a cerebral activity caused by the stimulations. As a representative apparatus for measuring the brain function, for example, an electroencephalography (EEG), a magnetoencephalograph (MEG), a functional magnetic resonance imaging (fMRI) apparatus, a functional near-infrared spectroscopy (fNIRS) apparatus, and a positron emission tomography (PET) apparatus have been known.

These apparatus measure an amount of the cerebral activity by detecting a physical amount such as a change of an electromagnetic wave (EEG and MEG) or a change of a blood flow rate (fMRI, fNIRS, and PET) generated by an activity of a nerve in an area of the brain in response to the stimulation presented to the human being. However, since these types of physical amounts generally have weak signals, they are significantly affected by various events such as a noise or a change of a cerebral activity that are not related to the presented stimulus.

Therefore, a measurement method is often used in which the stimulus to be presented to the subject is devised to extract the cerebral activity corresponding to the presented stimulus. For example, a method called a "block design" is often used in a brain function measurement using the fMRI method.

In the block design method, a state called a "rest block" in which the sensory stimuli is not presented to the subject and a state called a "task block" in which the sensory stimuli is presented to the subject are repeated in an alternate manner. Two types of images are then acquired, which include a functional brain imaging obtained in the rest period (i.e., an image in the state in which the sensory stimulus is not presented to the subject) and a functional brain imaging obtained in the task period (i.e., an image in the state in which the sensory stimulus is presented to the subject). Through comparison between the two types of images acquired in the above-mentioned manner, a functional brain imaging, on which a cerebral activity status in the state in which the sensory stimulation is presented to the brain is reflected, can be finally obtained.

According to "a course "Science of Sense and Perception" 1, Visual Sense I, Edited by Keizo Shinomori, Asakura Publishing Co., Ltd., ISBN 978-4-254-10631-2", it has been disclosed that, after the information is delivered to the brain from a sensory organ such as eyes and ears via a nerve system, a process of the sensory information in the brain is roughly divided into an initial stage of information processing (lower cognitive brain activity) and a later stage of information processing (higher cognitive brain activity).

To describe this with an example of the visual sense, the visual information is transformed from an optical signal into an electrical signal in a retina and delivered to an area of the cerebral cortex for processing an initial stage of the visual information, which is called a "primary visual cortex" (hereinafter, abbreviated as "V1"), via the nerve system. In the V1 area, local visual characteristics such as a slope of a line, a motion, a depth, and a color are detected from among image information included in the visual information. This process is called a "lower-order visual processing".

The information processed in the V1 is then delivered to a portion for performing a process for finally performing a "visual perception", which is called a "higher-order visual processing".

The "higher-order visual processing" is roughly divided into two processes.

One of the processes is for processing information on the motion or the depth by obtaining information that is required to perform a mutual action between a surrounding environment or object and the human being. These pieces of information are processed in the cerebral cortex called a "dorsal stream", and processed in an area assigned with a name such as medial temporal (MT), medial superior temporal (MST), and intraparietal areas (IPA).

The other of the processes is a function of recognizing an object or a person by collating information on the object or the person with a memory, which requires recognitions of color and detailed form including a three-dimensional structure. These pieces of information are processed in the cerebral cortex called a "ventral stream", and processed in an area assigned with a name such as fourth visual cortex (V4) and inferotemporal (IT).

A situation is assumed in which a lower-order processing function and a higher-order processing function of the brain on the visual sense are measured in a separate manner in the block design method described above.

For example, when an image of a face is presented to a subject, a lower-order visual cortex such as the V1 area and an area called a "fusiform gyrus (FG)" included in the IT area that uniquely responds to the image of the face from among the higher-order visual information processing, in response to a "task block" of showing the image of the face. In a "rest block" in which nothing is presented, none of the V1 and FG areas shows response. In general, the function of the brain with respect to certain stimulation is determined with a difference between the information obtained in the "task block" and the information obtained in the "rest block".

However, in this case, the lower-order visual information obtained in the V1 area and the higher-order visual information obtained in the FG area respond to the task in the task block (presentation of the image of the face to the subject) in a virtually simultaneous manner, and hence it is difficult to separately obtain the signals.

To cope with these problems, regarding a method of detecting a visual brain function by using the fMRI method, Japanese Patent Application Laid-Open No. 2007-190352 has disclosed the following method illustrated in FIGS. 9A, 9B and 9C. In this technique, two stimulations having a predetermined stimulation interval (ISI) are grouped as a pair, and this pair is sequentially presented with a constant stimulation period T by changing the stimulation interval (ISI) of the pair. The stimulation interval (ISI) of the pair of stimulations is gradually increased and then gradually decreased so that a temporal change of an activity in each area of the brain can be figured out with such an action as increasing the temporal resolution in an equivalent manner by shifting a timing for presenting the stimulation little by little. As a result, a time difference in the information processing between a fusiform face area (FFA) near the IT area that is supposed to process the information on the face and a visual cortex can be detected.

However, in the conventional technique described above, which can only be applied to the brain function measurement using the fMRI method, the timing for presenting the stimulation is changed by controlling the ISI, and hence a control of the stimulation to be presented becomes complicated.

Further, an image of a desired timing is obtained only two times in a period in which the ISI is increased and decreased one time from a measurement target area of the brain responding to the periodic stimulation. However, since the fMRI signal is weak, in order to obtain an image with a high S/N ratio, it is necessary to take multiple images and to perform an image processing such as averaging the obtained images.

In addition, if there are multiple desired timings, sampling of the above-mentioned processing needs to be performed as many times as needed, which may cause an increase of the imaging time or the data size.

### SUMMARY OF THE INVENTION

In view of the above-mentioned problems, the present invention is directed to a simply configured brain function measuring apparatus and a brain function measuring method that enable a measurement of a higher cognitive brain activity induced by a stimulation separately from a lower cognitive brain activity, when detecting a brain function by presenting the stimulation to a subject.

According to an exemplary embodiment of the present invention, there is provided a brain function measuring apparatus, including: a stimulation presenting unit configured to present multiple stimuli to a subject; and a brain function measuring unit configured to measure a brain function activity of the subject responding to the multiple stimulations. The multiple stimuli are adjusted to have no cognitive meaning when presented in a separate manner and to have a cognitive meaning when presented simultaneously as a combined stimulus. The stimulation presenting unit is configured to present the combined stimulus at regular intervals.

Further, according to an exemplary embodiment of the present invention, there is provided a brain function measuring method, including: presenting multiple stimulations to a subject; and measuring a brain function activity of the subject responding to the multiple stimulations. The multiple stimuli are adjusted to have no cognitive meaning when presented in a separate manner and to have a cognitive meaning when presented simultaneously as a combined stimulus. This presenting procedure includes presenting the combined stimulus at regular intervals.

According to the present invention, the brain function measuring apparatus and the brain function measuring method can be achieved which enable a measurement of a higher-order brain function activity induced by a stimulation separately from a lower-order brain function activity with a simple configuration when detecting a brain function activity by presenting the stimuli to a subject.

Further features of the present invention will become apparent from the following description of exemplary embodiments with reference to the attached drawings.

### BRIEF DESCRIPTION OF THE DRAWINGS

FIG. 1 is a cross-sectional view of a brain function measuring apparatus (functional magnetic resonance imaging (fMRI) apparatus) including a visual stimulation presenting apparatus for brain function measurement according to a first embodiment of the present invention.

FIGS. 2A, 2B, and 2C are diagrams illustrating image stimuli presented by the visual stimulation presenting apparatus for brain function measurement according to the first embodiment of the present invention.

FIGS. 3A, 3B, and 3C are diagrams illustrating specimens for visual stimulus used in the first embodiment of the present invention.

FIG. 4 is a graph showing a relation between the stimulation presentation and the BOLD response in the brain.

FIGS. 5A, 5B, 5C, 5D, 5E, and 5F are graphs showing a relation between the stimulation presentation and the BOLD response according to the first embodiment of the present invention.

FIG. 6 is a schematic diagram of an electroencephalography (EEG) apparatus including a visual stimulation presenting apparatus for brain function measurement according to a second embodiment of the present invention.

FIGS. 7A, 7B, 7C, 7D, 7E, and 7F are graphs showing a relation between the stimulation presentation and the brain wave response according to the second embodiment of the present invention.

FIG. 8 is a schematic diagram of a cerebral cortex used for describing a measurement method according to the second embodiment of the present invention.

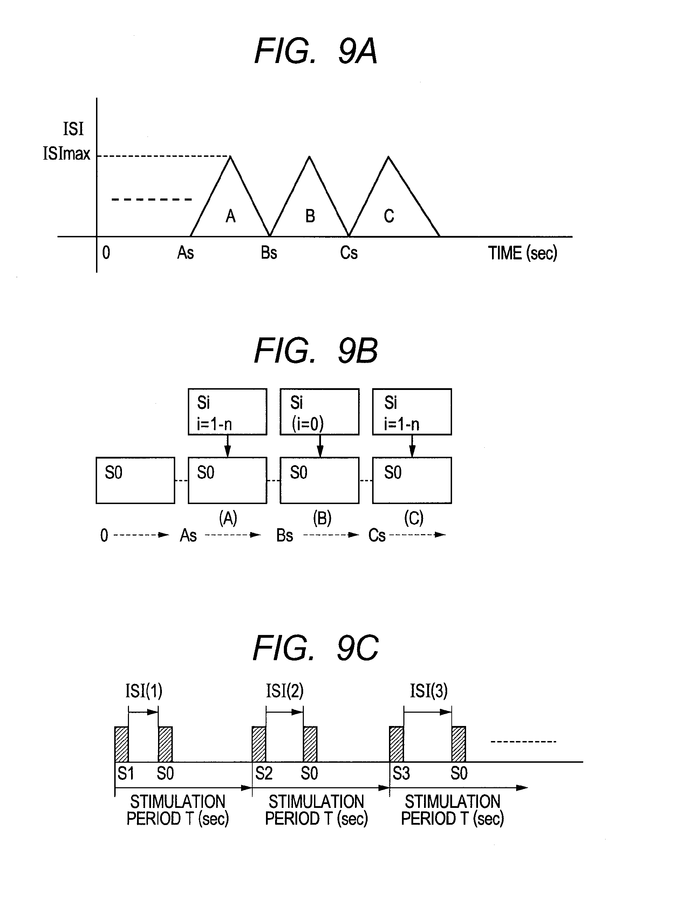

FIGS. 9A, 9B, and 9C are protocol diagrams for presenting a visual stimulus to a subject employed in a magnetic resonance imaging apparatus for measuring a brain function according to a conventional example.

### DESCRIPTION OF THE EMBODIMENTS

An exemplary embodiment of the present invention relates to a brain function measuring apparatus and a brain function measuring method for measuring a brain function of a subject by presenting a stimulus to the subject through an application of the present invention.

The embodiment of the present invention provides a stimulation presenting unit configured to present multiple stimulations as the stimulus to be presented to the subject. The embodiment further provides a brain function measuring unit configured to measure a brain function activity of the subject responding to the multiple stimulations, and a control unit configured to control the overall brain function measuring apparatus including the stimulation presenting unit and the brain function measuring unit. From multiple stimulations each having a different frequency component and having no cognitive meaning alone, a combined stimulation having a difference frequency between the different frequencies and having a cognitive meaning is generated at regular intervals for measurement, and a higher-order brain function activity responding to this combined stimulation is measured separately from a lower-order brain function activity.

That is, information having a higher-order cognitive meaning is superimposed when combining the multiple stimulations having different frequencies from each other and the information is modulated with a difference frequency between the different frequencies (beat frequency) so that the resultant information can be detected. With this configuration, by extracting and detecting a brain function activity responding to the beat frequency, a higher-order brain cognitive function activity can be detected without being affected by a measurement result of a lower-order brain function activity.

According to the above-mentioned configuration, a brain function activity in a higher-order visual area can be extracted separately from a brain function activity in a lower-order visual area by using multiple stimuli of simpler forms.

In the brain function measuring method and the brain function measuring apparatus according to this embodiment configured in the above-mentioned manner, specifically, a configuration can be adopted which has a function of detecting, with respect to arbitrary two stimulations modulated with different frequencies Xi [Hz] and Xj [Hz], respectively, from multiple stimulations to be presented to a subject (where i=1, 2, 3, ..., N, j=1, 2, 3, ..., N, i≠j, and N is the number of stimulations), a brain activity having a frequency component of Xi [Hz], Xj [Hz], and |Xi-Xj| [Hz].

With this configuration, a combined stimulation having the difference frequency |Xi-Xj| is generated from the two stimulations having the different frequency components of Xi and Xj. If information having a higher-order cognitive meaning is superimposed on this combined stimulation, the information is modulated with the difference frequency |Xi-Xj| (beat frequency), and therefore, a brain function activity corresponding to the beat frequency can be extracted and detected.

As a result, a component of the higher-order brain cognitive function activity generated by the presentation of the combined stimulation can be detected alone without being affected by a measurement result of the lower-order brain function activity, i.e., stimulations modulated with the frequencies Xi and Xj, respectively.

The stimulations to be presented can include at least one of a visual stimulation, an auditory stimulation, a haptic stimulation, an olfactory stimulation, a gustatory stimulation, and combinations thereof. With this configuration, the brain function activity generated by a combination of multimodal stimulations can be measured by measuring a cerebral activity having the frequency component of |Xi-Xj| described above.

An apparatus for measuring the cerebral activity can be at least one of a functional magnetic resonance imaging apparatus, a magnetoencephalograph, an electroencephalography, a near-infrared spectroscopy brain function measuring apparatus, a positron emission tomography apparatus, and combinations thereof. With this configuration, the higher-order cerebral activity with respect to the presented stimulation, which has conventionally been measured mainly by the fMRI apparatus, can be measured by other measuring apparatus and methods as described above.

(Embodiments)

Specific embodiments of the present invention are described below.

(First Embodiment)

A first embodiment of the present invention relates to an example of implementing the brain function measuring apparatus and the brain function measuring method by providing, in a functional magnetic resonance imaging (fMRI) apparatus, an apparatus that can present two visual stimuli to a subject as the multiple stimulations to the subject when measuring a brain function.

A functional magnetic resonance imaging (fMRI) apparatus is preferred in this embodiment because the functional magnetic resonance imaging (fMRI) apparatus can obtain an image of an active site in a brain accompanied by an onset of a brain function with high sensitivity and spatial resolution.

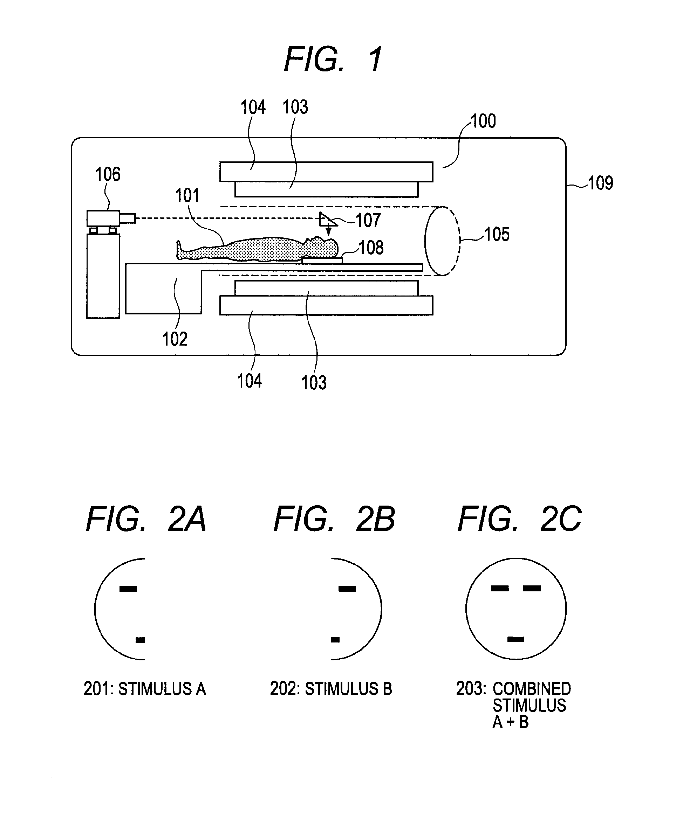

FIG. 1 illustrates a configuration example according to the first embodiment in which a visual stimulus presenting apparatus for brain function measurement, configured to present a specimen for visual stimulation to a subject in a selective manner, is provided in a functional magnetic resonance imaging apparatus configured to measure a brain function of a subject.

In the first embodiment, as illustrated in FIG. 1, a subject 101 lies down on a bed 102 provided in an MRI apparatus 100, which is then disposed in a cylindrical measurement unit (bore) 105 including a gradient magnetic field coil 103 and a superconducting magnet 104. A projector (stimulation presenting unit) 106 for presenting a visual stimulus to the subject 101 is provided outside the bore 105 in a direction of the feet of the subject 101. A picture or image stimulus is projected from the projector 106 onto a screen 107 that is located in front of eyes of the subject 101 in the bore 105. A coil (brain function measuring unit) 108 for detecting an MR signal is installed on the backside of the head of the subject 101, which detects an electromagnetic signal generated by a change of a cerebral blood flow accompanied by a neural activity of the subject 101. In this case, in order to secure a view in front of the subject 101 and to perform a highly sensitive brain function measurement in a visual cortex, i.e., the back of the head of the subject 101, the coil 108 to be used is a surface type radio frequency (RF) coil.

All these components are installed in a magnetically shielded room 109. The picture or image projected from the projector 106 is controlled by a computer (control unit, not shown) configured to control the overall apparatus, which is set outside the magnetically shielded room 109.

The projector 106 can alternatively be installed outside the magnetically shielded room 109 by providing a window for transmitting the projection picture or image on the magnetically shielded room 109.

In the first embodiment, for example, a visual stimulus A 201 illustrated in FIG. 2A, a visual stimulus B 202 illustrated in FIG. 2B, and a combined stimulus A+B 203 illustrated in FIG. 2C, which can be obtained by presenting the visual stimulus A 201 and the visual stimulus B 202 to the subject 101 in a simultaneous manner, are presented to the subject 101 as the stimulations. The visual stimulus A 201 and the visual stimulus B 202 are figures that has no cognitive meaning when presented alone, and positions of the visual stimulus A 201 and the visual stimulus B 202 to be presented are adjusted, respectively, to represent a figure such as a face of a human being when presented simultaneously as the combined stimulus A+B 203.

These stimuli are presented on the screen 107 provided in front of eyes of the subject 101 via the projector 106 while being controlled by the computer.

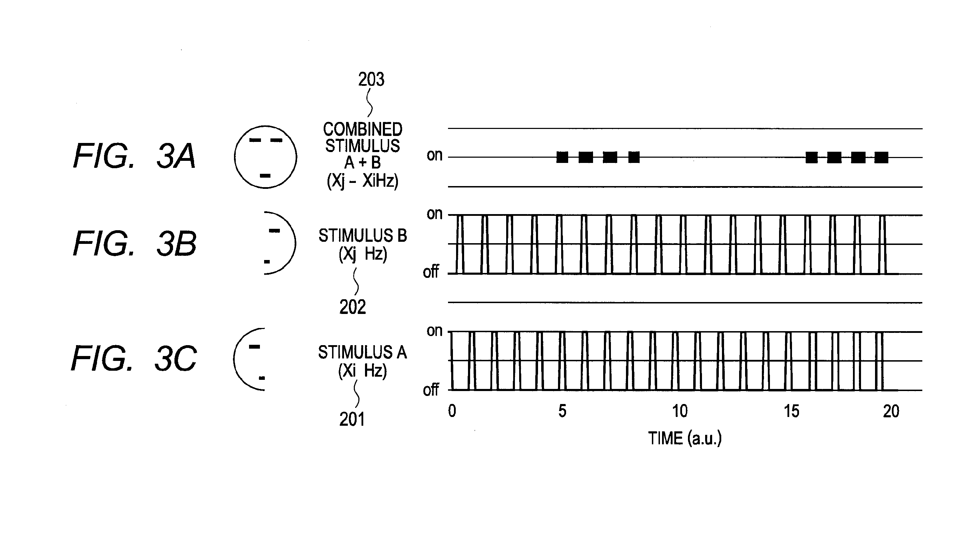

The visual stimulus A 201 is presented to the subject 101 while blinking (ON/OFF) with the frequency Xi [Hz] as illustrated in FIG. 3C. Although an ON/OFF time ratio of the blinking is set to 2:8 for simplicity, other values can also be used.

Similarly, the visual stimulus B 202 is presented to the subject 101 while blinking (ON/OFF) with the frequency Xj [Hz] as illustrated in FIG. 3B. In this case, the ON/OFF time ratio of the blinking is set to 2:8, where Xi≠Xj and |Xi-Xj|=0.1×Xi. In half a period (i.e., 1/(2×Xi) seconds) after starting a presentation of the visual stimulus A 201, a presentation of the visual stimulus B 202 is started.

In this embodiment, by presenting the stimuli to the subject 101 under the above-mentioned condition, the visual stimulus A 201 and the visual stimulus B 202 are presented to the subject 101 in a simultaneous manner as the combined stimulus A+B 203 with a predetermined period. That is, although the visual stimulus A 201 and the visual stimulus B 202 are initially presented to the subject 101 while blinking in an alternate manner, since Xi≠Xj, the combined stimulus A+B 203 becomes presented to the subject 101 with a predetermined period at a frequency as illustrated in FIG. 3A. This frequency is a difference frequency |Xi-Xj| [Hz] of the blinking frequency Xi of the visual stimulus A 201 and the blinking frequency Xj of the visual stimulus B 202. Therefore, by displaying the visual stimulus A 201 at the frequency Xi [Hz] and the visual stimulus B 202 at the frequency Xj [Hz] while blinking, the combined stimulus A+B 203 becomes presented to the subject 101 for every 1/|Xi-Xj| seconds.

This means that, by presenting the visual stimulus A 201 and the visual stimulus B 202, which have no cognitive meaning to the human being, to the subject 101 under the above-mentioned condition, the combined stimulus A+B 203 that cognitively means a "face" to the human being becomes presented to the subject 101 for every 1/|Xi-Xj| seconds.

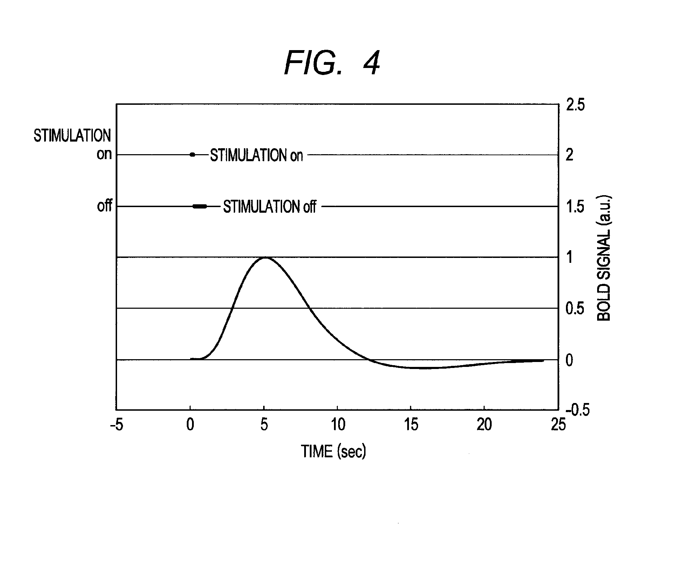

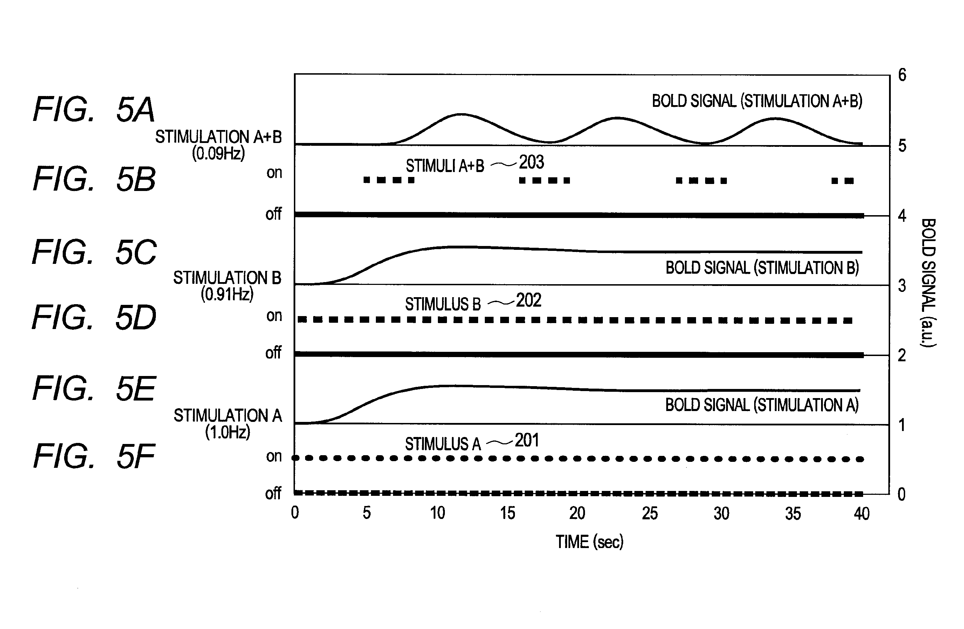

A result of measuring the cerebral activity of the subject 101 by the fMRI method when the visual stimulus A 201, the visual stimulus B 202, and the combined stimulus A+B 203 are presented to the subject 101 is shown in FIGS. 4 and 5A to 5F.

FIG. 4 is a graph showing a temporal change of the cerebral activity response, which is called a blood oxygenation level dependence (BOLD) signal used in the fMRI measurement when a pulsed visual stimulation is presented to the subject 101. When the pulsed visual stimulation is presented a single time, a nerve in the brain responds to the stimulation, and thus a smooth brain response signal such as the BOLD signal shown in FIG. 4 is obtained. This smooth response is generally called an fMRI signal (cerebral activity response) to a stimulation.

When the visual stimulus A 201 is repeatedly presented with a width of 0.2 seconds and Xi=1 Hz (1/Xi=1 second interval) as shown in FIG. 5F, an fMRI signal (BOLD signal to the visual stimulus A 201) is obtained as a waveform shown in FIG. 5E due to a temporal superposition of the signal. Further, when the visual stimulus B 202 is repeatedly presented with a width of 0.2 seconds and Xj≈0.91 Hz (1/Xj=1.1 second interval) with a delay of 0.5 seconds with respect to the visual stimulus A 201 as shown in FIG. 5D, the fMRI signal is obtained as a waveform shown in FIG. 5C. The visual stimulus A 201 and the visual stimulus B 202 are stimuli having no cognitive meaning as described above, and hence a portion of the brain responding to these stimulations is mainly the primary visual cortex V1.

Since the BOLD signals obtained by the visual stimulus A 201 and the visual stimulus B 202 have a time difference of about 0.5 seconds, when the primary visual cortex V1 is measured by the fMRI apparatus, both signals are detected in a state in which they are virtually overlapped without being separated.

On the other hand, the visual stimulus A 201 and the visual stimulus B 202 are modulated with frequencies Xi=1 Hz and Xj≈0.91 Hz, respectively, and hence a shift of the frequency is accumulated so that the combined stimulus A+B 203 is presented to the subject 101 as a beat signal with a period of |Xi-Xj|≈0.09 Hz (FIG. 5B).

Since the combined stimulus A+B 203 represents a figure of "face" having a cognitive meaning, the subject 101 recognizes this stimulus as a "face", and at this time, a response of the brain is generated at the fusiform face area (FFA) in the brain. The fMRI signal caused by this neural activity becomes a waveform shown in FIG. 5A, which oscillates at the frequency of 0.09 Hz.

In this manner, by presenting the visual stimulus A 201 having the frequency Xi=1 Hz and the visual stimulus B 202 having the frequency Xj=0.91 Hz to the subject 101 in a simultaneous manner, a response to the combined stimulus A+B 203 that has a cognitive meaning is generated in the brain (FFA) at the frequency of 0.09 Hz.

Through detection of a signal of this frequency, a higher-order visual cognitive signal can be detected in a separate manner without being affected by a lower-order visual cognitive brain signal generated as a primary vision.

It has been known that, when a pulsed sensory stimulus is presented to a subject in a repeated manner, an intensity of a response in the brain is decreased gradually due to an adaptation effect. In this case, a magnitude of the fMRI signal is decreased accordingly. In the cases of the visual stimulus A 201 and the visual stimulus B 202 described above, also, since the magnitude of the fMRI signal are repeatedly presented to the subject, they may be gradually decreased.

However, the combined stimulus A+B 203, which oscillates at the frequency of |Xi-Xj| Hz, can adjust the frequency |Xi-Xj| to the extent that a decrease of a signal level due to the adaptation effect can be reduced enough, and hence the higher-order visual cognitive signal can be separately detected at this frequency.

Although the functional magnetic resonance imaging (fMRI) apparatus is employed in this embodiment, a similar measurement can also be performed by using a positron emission tomography (PET). In this case, the subject is placed in a PET apparatus instead of the fMRI apparatus to perform the same measurement (not shown).

(Second Embodiment)

In a second embodiment of the present invention, the brain function measuring apparatus and the brain function measuring method are implemented by providing, in an electroencephalography (EEG) apparatus, an apparatus that can present two visual stimuli to a subject as multiple stimulations to be presented to the subject when measuring a brain function. In the case of using the electroencephalography apparatus, an electrical signal generated by a neural activity in the brain accompanied by an onset of a brain function can be measured with high sensitivity and high temporal accuracy.

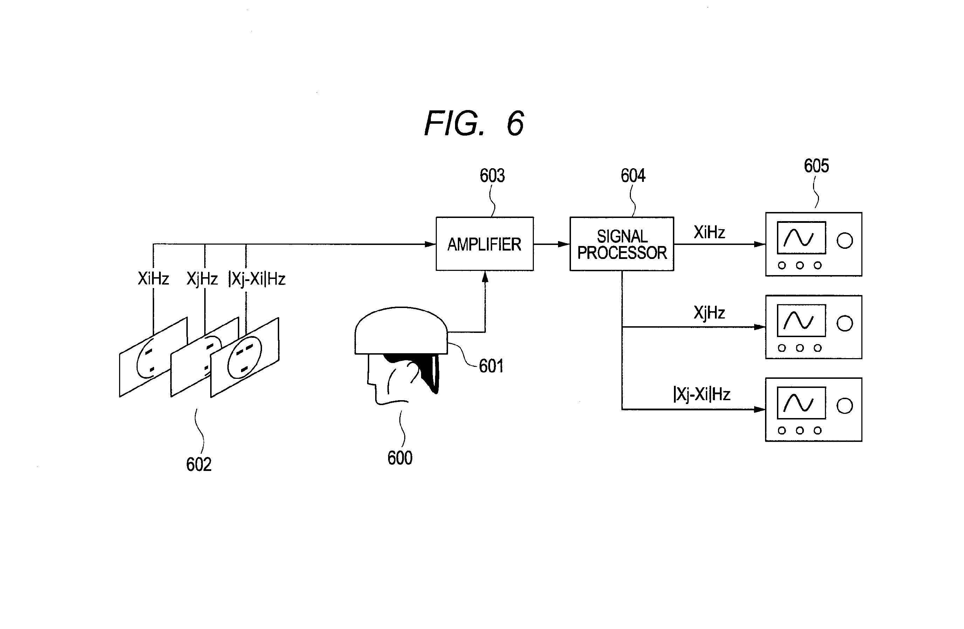

FIG. 6 is a diagram illustrating a configuration example in which a visual stimulation presenting apparatus for brain function measurement configured to present a specimen for visual stimulus to a subject in a selective manner is provided in a brain function measuring apparatus for measuring a brain function of a subject according to the second embodiment.

In this embodiment, as illustrated in FIG. 6, a subject 600 wears an electroencephalography apparatus 601 on the head, and in this state, a visual stimulus 602 is presented to the subject 600. The visual stimulus 602 can be presented to the subject 600 by projecting a projector image or picture on a screen, in the same manner as in the first embodiment, or by using a CRT or the like (not shown).

Through the presentation of the visual stimulus 602, an electrical signal generated by a neural activity in the brain of the subject 600 is detected by the electroencephalography apparatus 601. The detected electrical signal is amplified by an amplifier 603, and only a predetermined frequency signal is extracted by a signal processor 604 from the amplified electrical signal. The extracted frequency signal is then displayed on a signal display apparatus.

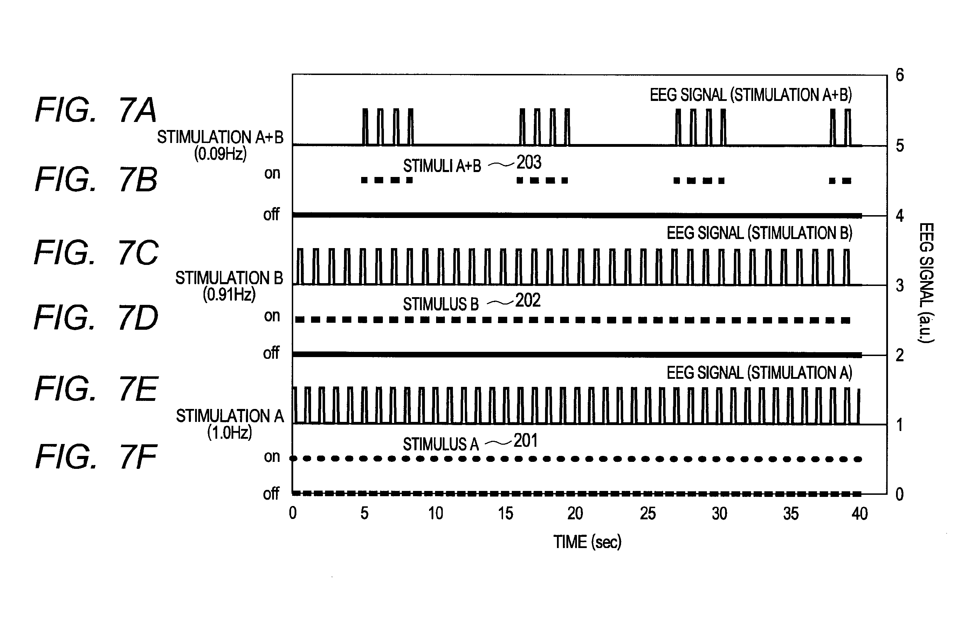

A measurement result obtained by the EEG apparatus when the same stimulations as used in the first embodiment are used as the visual stimulus to be presented to the subject 600 is shown in FIGS. 7A to 7F.

When the visual stimulus A 201 is repeatedly presented with a width of 0.2 seconds and Xi=1 Hz (1/Xi=1 second interval) as shown in FIG. 7F, an EEG signal is obtained as a waveform shown in FIG. 7E. Since the EEG signal is obtained by directly detecting the electrical signal generated by the neural activity in the brain, there is no time delay unlike the BOLD signal used in the fMRI measurement. When a pulsed stimulation is presented, the EEG signal is obtained as a pulsed response as shown in FIG. 7E.

Further, when the visual stimulus B 202 is repeatedly presented with a width of 0.2 seconds and Xj≈0.91 Hz (1/Xj=1.1 second interval) with a delay of 0.5 seconds with respect to the visual stimulus A 201 as shown in FIG. 7D, the EEG signal is obtained as a waveform shown in FIG. 7C.

The visual stimulus A 201 and the visual stimulus B 202 can be stimulations having no cognitive meaning as described above, and hence a portion of the brain responding to these stimulations is mainly the primary visual cortex V1.

On the other hand, since the visual stimulus A 201 and the visual stimulus B 202 are modulated with frequencies Xi=1 Hz and Xj≈0.91 Hz, respectively, a shift of the frequency is accumulated so that the combined stimulus A+B 203 is presented to the subject 600 as a beat signal with a period of |Xi-Xj|≈0.09 Hz (FIG. 7B).

Since the combined stimulus A+B 203 represents a figure of "face" having a cognitive meaning, the subject 600 recognizes this stimulation as a "face", and at this time, a response of the brain is generated at the fusiform face area (FFA) in the brain. The EEG signal caused by this neural activity becomes a waveform shown in FIG. 7A, which is obtained for every 1.1 seconds (frequency of 0.09 Hz). In this manner, by presenting the visual stimulus A 201 having the frequency Xi=1 Hz and the visual stimulus B 202 having the frequency Xj=0.91 Hz to the subject 600 in a simultaneous manner, a response to the combined stimulus A+B 203 that has a cognitive meaning is generated in the brain (FFA) at the frequency of 0.09 Hz.

Through detection of a signal of this frequency, a higher-order visual cognitive signal can be detected in a separate manner without being affected by a lower-order visual cognitive brain signal generated as a primary vision.

It is said that the EEG measurement is inferior to the fMRI measurement in terms of the spatial resolution. However, multiple electrodes can be used on the head of the subject in the EEG measurement, and the signals can be detected with a focus on electrical signals generated by neural activities near the electrodes.

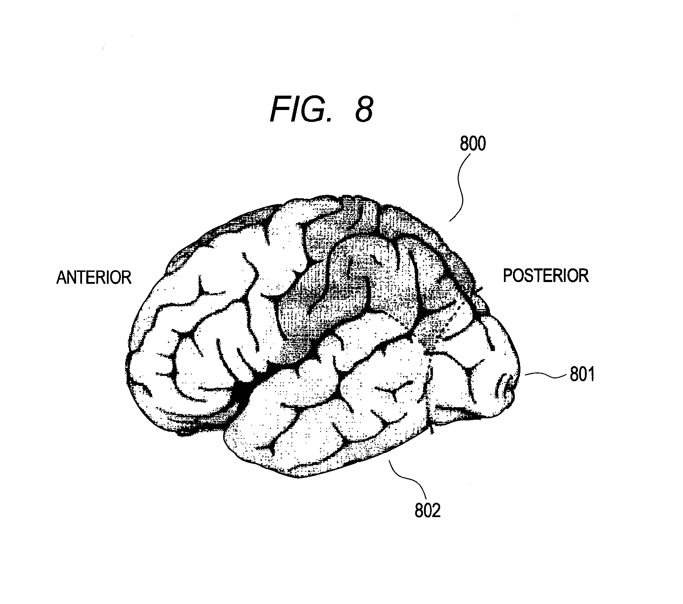

FIG. 8 is a schematic diagram of a cerebral cortex 800 of the left side of the brain of a human being. The primary visual cortex (V1) 801 is located on the posterior side of the cerebral cortex 800, and the fusiform face area (FFA) is located near an area 802 that is called the FG on the temporal area.

Therefore, when detecting signals on the visual stimulus A 201, the visual stimulus B 202, and the combined stimulus A+B 203, the signals can be detected with higher accuracy by disposing the EEG electrodes on the scalp near, A: primary visual cortex V1, B: primary visual cortex V1, and A+B: FG area, respectively.

Although a case of using the EEG measuring apparatus is described in this embodiment, the same measurement can be performed by using a magnetoencephalograph (MEG). In this case, the subject wears a magnetoencephalograph apparatus (not shown) on the head instead of the electroencephalography apparatus 601 for the EEG measurement. A measurement result can be obtained as the virtually same signal as obtained in the second embodiment using the EEG apparatus. In this case, among detecting elements in detecting devices of multiple MEG apparatus, signals respectively obtained from a detecting element located on the scalp near the primary visual cortex V1 of the cerebral cortex and a detecting element located on the scalp near the FG area are analyzed. With this operation, electromagnetic signals generated in the brain by the visual stimulus A 201, the visual stimulus B 202, and the combined stimulus A+B 203 can be separated.

Further, a similar measurement can be performed using a functional near-infrared spectroscopy (fNIRS) apparatus. When using the fNIRS apparatus, a signal detector (not shown) is worn by the subject on the head for measurement in the same manner as the EEG according to the second embodiment. The detector can be disposed in contact with the scalp near the primary visual cortex V1 and the FG area in the same manner as the EEG. However, since the fNIRS detects a BOLD signal, a relation between a presented stimulation and an obtained detection signal becomes similar to the waveform obtained using the fMRI in the first embodiment (FIGS. 5A to 5F).

In the first and second embodiments described above, examples of measuring the lower cognitive brain activity (no cognitive cerebral activity) and the higher cognitive brain activity (cognitive cerebral activity) separately from each other by using multiple visual stimulations are described.

However, in the brain function measuring apparatus and the brain function measuring method according to the present invention, the stimulation to be used is not limited to the visual stimulation. A similar measurement can be performed by using any one of the auditory stimulation, the haptic stimulation, the olfactory stimulation, and the gustatory stimulation. Further, a similar measurement can be performed by using a combination of different types of stimulations (multimodal stimulation), such as a combination of the visual stimulation and the auditory stimulation or a combination of the visual stimulation and the haptic stimulation. Also in the case of using the multimodal stimulation, if stimulations forming the multimodal stimulation are modulated with different frequencies from each other, the lower-order brain function activities generated by the respective stimulations and the higher-order brain function activity generated by a combination of the stimulations can be measured separately from each other.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.
A brain function activity of a subject is measured using a brain function measuring unit by presenting multiple stimulations, which are adjusted to have no cognitive meaning when presented in a separate manner and to have a cognitive meaning when presented simultaneously as a combined stimulation, to the subject as the combined stimulation at regular intervals with a control of a control unit, thus measuring a higher cognitive brain activity responding to the combined stimulation separately from a lower cognitive brain activity.

## Claims

1. A brain function measuring apparatus, comprising:
a stimulation presenting unit configured to present multiple stimuli which function as multiple stimulations to a subject; and
a brain function measuring unit configured to measure a brain function activity of the subject responding to the multiple stimulations, wherein:
the multiple stimulations are adjusted to have no cognitive meaning when presented in a separate manner and to have a cognitive meaning when presented simultaneously as a combined stimulation; and
the stimulation presenting unit is configured to present the combined stimulation at regular intervals.

2. The brain function measuring apparatus according to claim 1, wherein:
the multiple stimulations are modulated with different frequencies from each other; and
the combined stimulation is configured to have a difference frequency between the different frequencies.

3. The brain function measuring apparatus according to claim 1, wherein the multiple stimulations comprise at least one of a visual stimulation, an auditory stimulation, a haptic stimulation, an olfactory stimulation, a gustatory stimulation, and combinations thereof.

4. The brain function measuring apparatus according to claim 1, wherein the brain function measuring unit comprises at least one of a functional magnetic resonance imaging apparatus, a magnetoencephalograph, an electroencephalography, a near-infrared spectroscopy brain function measuring apparatus, a positron emission tomography apparatus, and combinations thereof.

5. A brain function measuring method, comprising:
presenting multiple stimuli which function as multiple stimulations to a subject; and
measuring a brain function activity of the subject responding to the multiple stimulations, wherein:
the multiple stimulations are adjusted to have no cognitive meaning when presented in a separate manner and to have a cognitive meaning when presented simultaneously as a combined stimulation; and
the presenting comprises presenting the combined stimulation at regular intervals.

6. The brain function measuring method according to claim 5, wherein:
the multiple stimulations are modulated with different frequencies from each other; and
the combined stimulation is configured to have a difference frequency between the different frequencies.

7. The brain function measuring method according to claim 5, wherein the multiple stimulations comprise at least one of a visual stimulation, an auditory stimulation, a haptic stimulation, an olfactory stimulation, a gustatory stimulation, and combinations thereof.

8. The brain function measuring method according to claim 5, wherein the measuring comprises measuring the brain function activity of the subject by using at least one of a functional magnetic resonance imaging apparatus, a magnetoencephalograph, an electroencephalography, a near-infrared spectroscopy brain function measuring apparatus, a positron emission tomography apparatus, and combinations thereof.
